## Europäisches Patentamt

## European Patent Office

## Office européen des brevets

(19)

(11) Veröffentlichungsnummer: **0 209 053**
A2

(12) **EUROPÄISCHE PATENTANMELDUNG**

(21) Anmeldenummer: **86109297.1**

(22) Anmeldetag: **08.07.86**

(51) Int. Cl.⁴: **G 10 K 9/12**

(30) Priorität: **18.07.85 DE 3525641**
**23.07.85 DE 8521196 U**

(43) Veröffentlichungstag der Anmeldung: **21.01.87**
**Patentblatt 87/4**

(84) Benannte Vertragsstaaten: **CH DE FR GB IT LI NL SE**

(71) Anmelder: **Eisenmenger, Wolfgang Prof. Dr.,**
**Landhausstrasse 7, D-7140 Ludwigsburg (DE)**

(72) Erfinder: **Eisenmenger, Wolfgang Prof. Dr.,**
**Landhausstrasse 7, D-7140 Ludwigsburg (DE)**

(74) Vertreter: **Wilhelm, Hans-Herbert, Dr.-Ing. et al, Wilhelm**
**& Dauster Patentanwälte Hospitalstrasse 8,**
**D-7000 Stuttgart 1 (DE)**

(54) Verfahren und Einrichtung zur berührungsfreien Zertrümmerung von Konkrementen im Körper von Lebewesen.

(57) Bei einem Verfahren bzw. bei einer Einrichtung zur berührungsfreien Zertrümmerung von Konkrementen im Körper von Lebewesen erzeugt eine Quelle Stoßwellen, die auf das Konkrement fokussiert werden und dieses zerstören. Zur Vermeidung von Gewebeverletzungen in dem behandelten Körper wird das Profil der Druckamplituden der zu fokussierenden Stoßwellen örtlich nach außen hin abgesenkt. Eine weitere, verstärkende Möglichkeit, Gewebeverletzungen zu vermeiden, besteht darin, daß die Druckamplituden der Stoßwellen zeitlich zuerst schnell erhöht und dann wieder verringert werden. Bei einer Einrichtung zur Durchführung des Verfahrens wird eine Metallmembran von einer von Stromimpulsen beaufschlagten Leiteranordnung zu Stößen angeregt, die über entsprechende Kopplungskörper und Fokussiereinrichtungen als Stoßwellen auf das zu zerstörende Konkrement hingeführt werden. Zur Absenkung des Profils der Druckamplituden im Randbereich der Stoßwellen wird dabei zum Beispiel die Wicklungsdichte der Leiteranordnung zum Randbereich hin verringert oder die Drahtstärke der einzelnen Windungen der Leiteranordnung zum Randbereich hin vergrößert. Dieses Verfahren ist allgemein auf sämtliche Systeme zur Erzeugung und Fokussierung von Stoßwellen anwendbar.

Verfahren und Einrichtung zur berührungsfreien Zertrümmerung
von Konkrementen im Körper von Lebewesen

===================================================================

Die Erfindung betrifft ein Verfahren und eine Einrichtung zur
berührungsfreien Zertrümmerung von Konkrementen im Körper von
Lebewesen unter Zuhilfenahme von Stoßwellen, die auf das
Konkrement fokussiert sind.

Derartige Verfahren und Einrichtungen sind aus der deutschen
Offenlegungsschrift 33 12 014 und der nicht vorveröffentlichten
deutschen Patentanmeldung 34 43 295 bekannt. Dort ist eine
Leiteranordnung vorgesehen, die induktive Eigenschaften aufweist und als spiralförmige Spule oder als aus einem Flachband
gewickelte Spule ausgebildet ist und die Form einer Kugelkalotte hat. Bei Beaufschlagung dieser Spule mit einem Stromstoß, beispielsweise durch eine Kondensatorentladung über eine
Funkenstrecke, werden in einer Metallmembran, die von der Spule
durch eine dünne Isolierschicht getrennt ist, entgegengerichtete Ströme induziert, die zur Abstoßung dieser Membran führen.
Da die Membran in Kontakt mit einem Übertragungsmedium steht,
werden hierdurch Stoßwellen abgegeben, die aufgrund der kugelkalottenförmigen Ausbildung dieser Membran fokussiert werden.
Zur Vermeidung von Beugungen und Reflexionen, wie sie an dem
sich an die Metallmembran anschließenden Gehäuseteil ergeben
können, kann die Abstrahlseite der Metallmembran als kegelstumpfförmiger Hohlraum ausgebildet sein, in dem sich das Übertragungsmedium befindet.

Andere bekannte Einrichtungen arbeiten mit Unterwasserfunkenstrecken zur Druckwellenerzeugung und mit elliptischen Spiegeln
zur Fokussierung der Stoßwellen auf das Konkrement. Ebenfalls
ist bekannt, zur Stoßwellenerzeugung piezoelektrische Elemente
oder magnetostriktive Elemente zu verwenden.

Bei der Fokussierung von Druckstößen, die bei sämtlichen bekannten Vorgehensweisen notwendig ist, ist jedoch aus physikalischen Gründen das Auftreten von Unterdruckpulsen nicht völlig zu vermeiden. Überschreiten dabei diese Unterdruckpulse eine gewisse Stärke, so führt dies im Körper des behandelten Lebewesens, insbesondere in dem von den Stoßwellen durchdrungenen Gewebe zu Kavitation, d.h. zu mikroskopischen Aufreißungen und damit zu Gewebeverletzungen.

Aufgabe der Erfindung ist es, das eingangs genannte Verfahren und die eingangs genannte Einrichtung so weiterzubilden, daß Gewebeschädigungen nicht mehr auftreten. Dazu ist es notwendig, die auf den Einfluß von Randbeugungswellen des fokussierenden Systems zurückzuführenden Unterdruckpulse zu vermeiden.

Gelöst wird die Aufgabe erfindungsgemäß dadurch, daß bei einem Verfahren der eingangs genannten Art das Profil der Druckamplituden der Stoßwellen örtlich nach außen hin abgesenkt wird. Durch eine derartige Reduktion der Randschärfe der Stoßwellen wird eine wesentliche Abschwächung der Randbeugungswellen und damit der die Gewebeschädigungen hervorrufenden Unterdruckpulse erreicht. Gleichzeitig wird durch eine derartige Absenkung des Profils der Druckamplituden der Stoßwellen die Entstehung und Ausbildung der Stoßwellen wesentlich verbessert.

Bei einer besonders vorteilhaften Weiterbildung der Erfindung werden die Druckamplituden der Stoßwellen zeitlich zuerst schnell erhöht und dann wieder verringert. Mit Hilfe einer derartigen dreiecksförmigen Impulsform der Druckamplitude wird erreicht, daß die Möglichkeit des Auftretens von Unterdruckpulsen noch weiter verringert wird. Das zeitlich dreiecksförmige Verhalten der Druckamplituden verstärkt also die Wirkung der örtlich nach außen hin stattfindenden Absenkung der Druckamplituden.

Besonders vorteilhaft ist es, die Druckamplituden dadurch zu beeinflussen, daß auf die Anregungsdichte oder Anregungsstärke der Stoßwellen oder auf beide Parameter gleichzeitig eingewirkt wird. Dabei ist es unerheblich, wie die Stoßwellen erzeugt werden, ob es sich also bei dem System zur Stoßwellenerzeugung um ein spulenförmiges, ein piezoelektrisches, ein magnetostriktives System, oder ein System mit Unterwasser-Funkenentladung und Ellipsoid-Fokussierung handelt, da bei sämtlichen Systemen deren Anregungsdichte und Anregungsstärke auf irgend eine Art und Weise beeinflußbar ist.

Bei einer erfindungsgemäßen Einrichtung zur Durchführung des erfindungsgemäßen Verfahrens sind Mittel vorgesehen, um eine örtlich nach außen hin verlaufende Absenkung sowie ggf. eine zeitlich zuerst schnelle Erhöhung und darauffolgende Verringerung der Druckamplituden der Stoßwellen zu bewerkstelligen. Dabei kann es sich bei den Mitteln wieder um spulenförmige, piezoelektrische oder magnetostriktive Elemente oder um die Stoßwelle fokussierende Elemente handeln sowie um Flüssigkeitsfunkenentladungen oder Impulsgeneratoren.

Eine besonders vorteilhafte Ausgestaltung der erfindungsgemäßen Einrichtung umfaßt eine Metallmembran, die auf ihrer einen Seite durch eine dünne Isolierschicht von einer flachen Leiteranordnung getrennt ist und auf ihrer anderen Seite gegen ein Übertragungsmedium gedrückt wird, wobei die Leiteranordnung als Kugelkalottenfläche ausgestaltet ist und mit einem elektrischen Impuls bestimmter Stärke und Zeitdauer beaufschlagt werden kann. Damit bei einer derartigen Einrichtung bei deren Betrieb keine Unterdruckpulse entstehen, also eine Gewebeschädigung vermieden wird, kann entweder die Windungsdichte der Leiteranordnung nach außen hin verringert werden, die Leiterstärke der Leiteranordnung nach außen hin vergrößert werden oder die Leiteranordnung aus Einzelwindungen- bzw. spulen bestehen. Eine Kombination dieser Möglichkeiten ergibt eine Verstärkung des Randunschärfeeffektes, so daß damit die Möglichkeit von Gewebeverletzungen noch weiter verringert wird.

Bei der eingangs erwähnten Einrichtung nach der DE-OS 33 12 014
grenzt eine Metallmembran an eine kugelkalottenförmig
ausgebildete Leiteranordnung an. Die Leiteranordnung ist in der
Form einer spiralförmig aus Draht gewickelten Spule aufgebaut.
Vom Zentrum der Spule und vom Außenrand der Spule ist jeweils
ein Anfangsstück des Spulendrahtes herausgeführt.

Die Herstellung derartiger Membran-Spulen-Anordnungen ist
bisher mit großen Schwierigkeiten verbunden und wird größtenteils in Handarbeit durchgeführt. Insbesondere die starken Biegungen im Zentrum der spiralförmigen Spule führen oft zu Beschädigungen des Isolierlacks des Spulendrahtes und damit, aufgrund der erhöhten Durchschlags- bzw. Kurzschlußgefahr, zur
Unbrauchbarkeit der Spule. Ebenso entstehen aufgrund der hohen
Beanspruchung der Metallmembran, insbesondere in ihrem Zentrum
Ermüdungsbrüche, was wiederum die Unbrauchbarkeit der gesamten
Spulen-Membran-Anordnung zur Folge hat. Insgesamt ist es deshalb bisher nicht möglich, die bekannten Spulen-Membran-Anordnungen mit hohen Lebensdauern auszustatten.

Es ist daher eine weitere Aufgabe der Erfindung, die Standzeit,
also die Lebensdauer der bekannten Membran-Spulen-Anordnung zu
erhöhen.

Gelöst wird diese Aufgabe dadurch, daß bei einer gattungsgemäßen Anordnung der innere Anfangspunkt des Leiters außerhalb
des Zentrums der Spule liegt. Dadurch wird erreicht, daß im
Wickelzentrum der Spule aufgrund des größeren Krümmungsradius
des Leiters keine scharfen Ecken und Kanten entstehen und der
Isolierlack des Leiters nicht mehr beschädigt wird. Der Abstand
des inneren Anfangspunktes der Spule vom Zentrum der Spule ist
u. a. vom Querschnitt des verwendeten Leiterdrahtes abhängig
und kann durch entsprechende Wickelversuche optimiert werden.

Bei einer besonders vorteilhaften Weiterbildung der Erfindung
besitzt die Membran Dehnungsfalten. Diese können kreisförmig,
radial oder spiralförmig angeordnet sein. Derartige Dehnungsfalten haben zur Folge, daß sich die Membran besser ausdehnen
kann und dadurch die Gefahr von Brüchen der Membran verringert
wird.

Weitere vorteilhafte Ausgestaltungen und Weiterbildungen der
Erfindung ergeben sich aus den Unteransprüchen sowie aus der
nachfolgenden Beschreibung der Zeichnung, in der Ausführungsbeispiele der Erfindung dargestellt sind. Es zeigen:

Fig. 1   eine schematische Darstellung eines Ausführungsbeispieles einer Einrichtung zur berührungsfreien Zertrümmerung von Konkrementen im Körper von Lebewesen,

Fig. 2   ein Diagramm, in dem das Profil der Druckamplitude p
         über dem radialen Abstand r von der Systemachse
         aufgetragen ist,

Fig. 3   ein weiteres Diagramm, in dem die Druckamplitude p
         über der Zeit t aufgetragen ist,

Fig. 4   einen Ausschnitt aus der Einrichtung gemäß Fig. 1
         zur Darstellung einer weiteren Ausführungsform der
         erfindungsgemäßen Einrichtung,

Fig. 5   eine schematische Seitenansicht einer ebenen Membran-
         Spulen-Anordnung, die bei einer elektromagnetisch
         wirkenden Einrichtung zur Erzeugung von Stoßwellen,
         insbesondere zur Zertrümmerung von Konkrementen im
         Körper von Lebewesen einsetzbar ist,

Fig. 6   eine schematische Seitenansicht einer kugelkalottenförmigen Membran-Spulen-Anordnung, die bei einer Einrichtung nach Fig. 1 oder 4 einsetzbar ist,

Fig. 7 eine schematische Draufsicht auf eine der Spulen der
    Fig. 5 oder 6,

Fig. 8
bis 10 schematische Draufsichten auf eine der Metallmembranen
    der Fig. 5 oder 6,

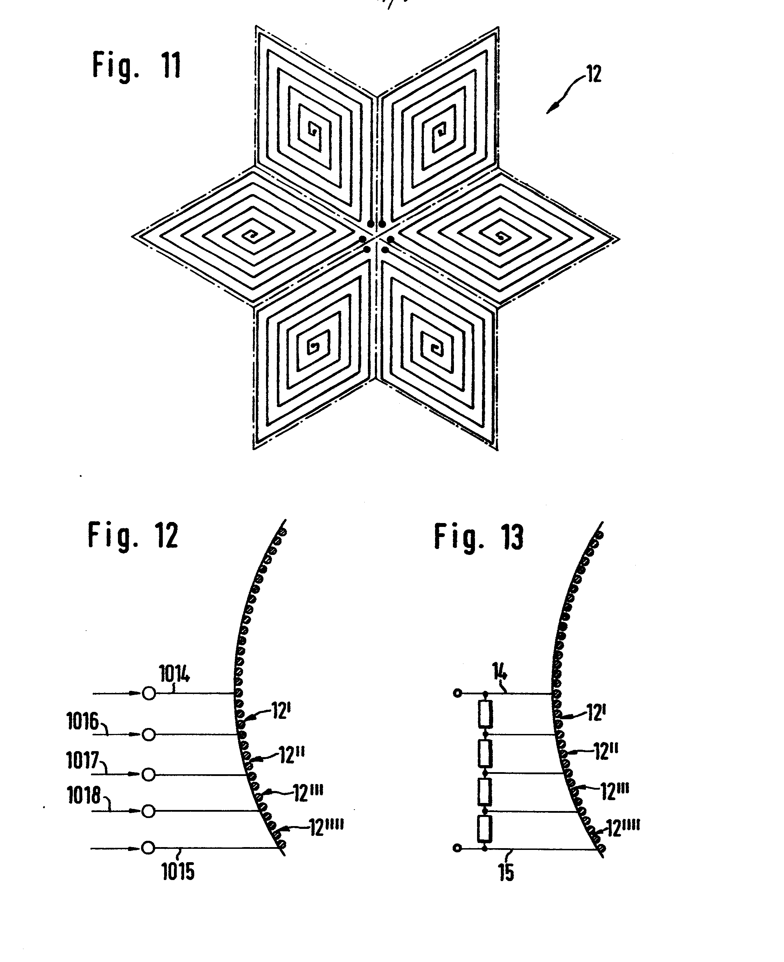

Fig. 11 eine Ausführungsform mit einer sternförmig ausgebildeten Spulenanordnung, die durch die Kombination von
    sechs rhombischen Spulen erreicht wird,

Fig. 12
und 13 mögliche Ausführungsformen der Merkmale der Ansprüche,

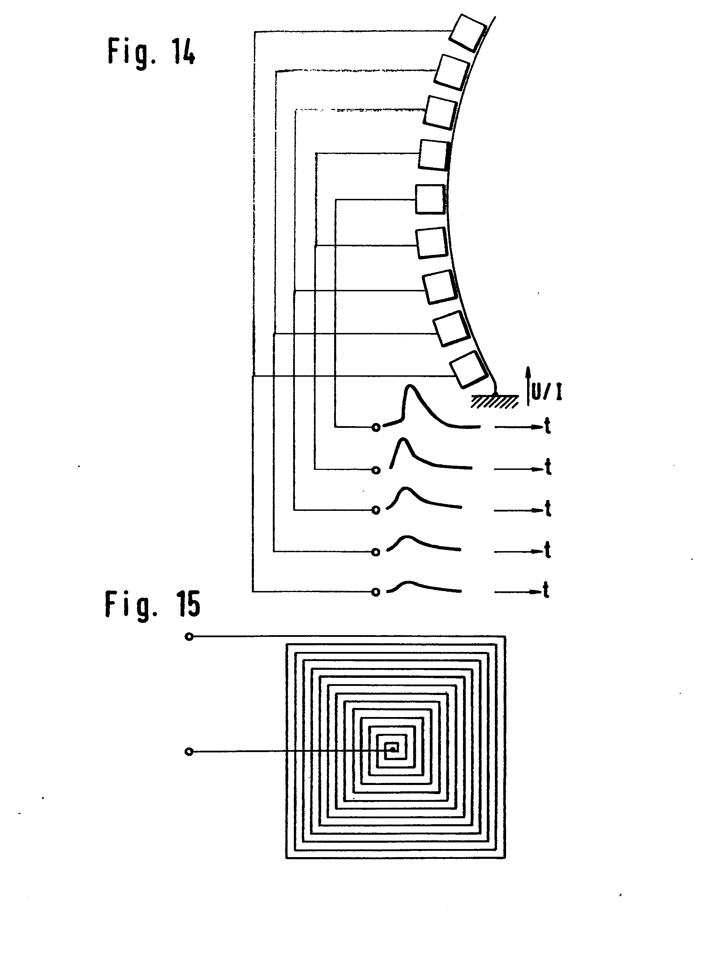

Fig. 14 eine Ausführungsform mit den Merkmalen der Ansprüche 8
    und 9, bei der die einzelnen Elemente mit unterschiedlichen Impulsen beaufschlagt werden und

Fig. 15 eine Spulenausbildung für eine Anordnung mit einem
    Ausbreitungskonus mit quadratischem Querschnitt.


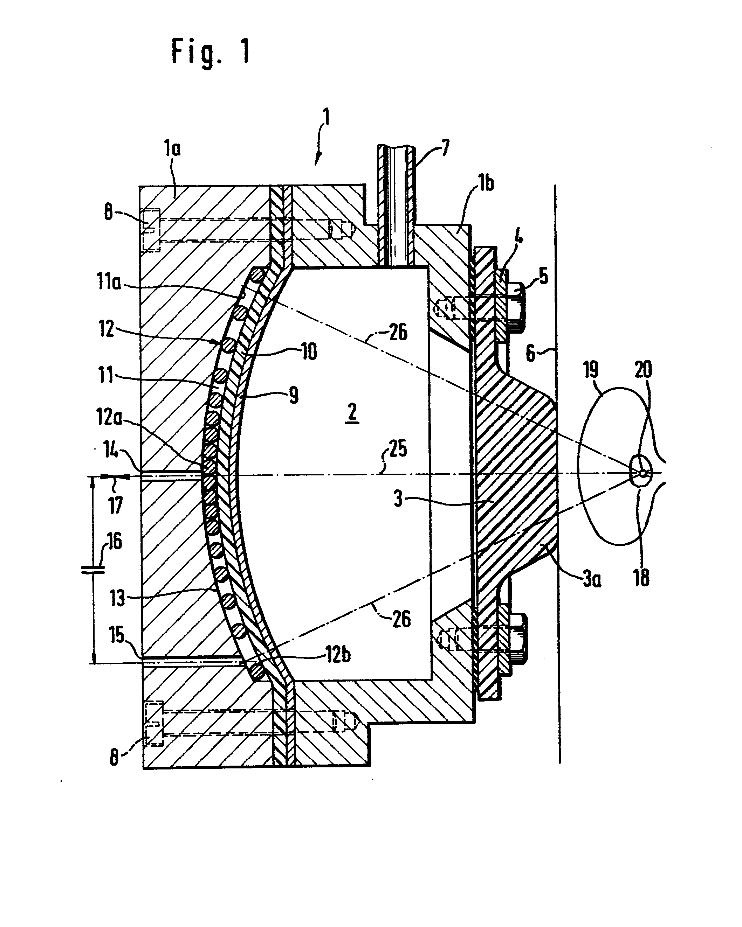

In der Fig. 1 ist schematisch ein Gehäuse 1 gezeigt, das aus den beiden Gehäuseteilen 1a und 1b besteht, von denen der Gehäuseteil 1b hohl ist und mit einer Flüssigkeit, beispielsweise mit Wasser gefüllt ist. Der Hohlraum 2 des Gehäuseteiles 1b ist durch eine elastische Abdeckung 3 abgeschlossen, die über einen Befestigungsring 4 mit Schrauben 5 dicht an den Gehäuseteil angesetzt ist. Die Abdeckung 3 weist einen pufferartigen Ansatz 3a auf, der dicht und u. U. unter Zwischenfügung einer Schicht einer Kopplungsflüssigkeit an dem zu behandelnden Körperteil anliegt, dessen Begrenzung 6 nur durch eine Grenzlinie angezeigt ist. Die Abdeckung 3 besteht beispielsweise aus einem gummielastischen Material. Der Hohlraum 2 innerhalb des Gehäuseteiles 1b steht über den Anschlußstutzen 7 mit einer nicht gezeigten Druckquelle in Verbindung, die dafür sorgt, daß in dem Hohlraum stets ein gewisser statischer Überdruck in der Flüssigkeit erhalten bleibt. Natürlich könnte auch, da es nur auf den Differenzdruck ankommt, im Raum vor der kugelkalottenförmigen Außenseite 11 und hinter der Membran 9 ein gewisser Unterdruck gegenüber dem Druck im Hohlraum 2 herrschen. Auch dann würde die Membran 9 fest gegen die Leiteranordnung 12 gedrückt.

Der Gehäuseteil 1b und der Gehäuseteil 1a sind über Schrauben 8 zusammengefügt. Dabei werden zwischen beiden Gehäuseteilen eine Metallmembran 9 und eine aus elektrisch isolierendem Material bestehende Isolierfolie 10 eingespannt. Dabei werden die beiden zuletzt genannten Bauteile 9 und 10 unter dem Druck der Flüssigkeit in dem Hohlraum 2 eng an die kugelkalottenförmige Außenseite 11 des Gehäuseteiles 1a angedrückt, die beim gezeigten Ausführungsbeispiel von der äußeren Begrenzung einer Leiteranordnung 12 gebildet wird, die ihrerseits im wesentlichen aus einer aus elektrisch leitfähigem Draht, beispielsweise aus Kupferdraht aufgebauten Spule besteht. Dabei ist es auch möglich, anstatt oder zusätzlich zum Überdruck im Hohlraum 2, mittels entsprechender Anordnungen einen Unterdruck auf der dem Hohlraum 2 gegenüberliegenden Seite der Membran 9 bereitzustellen und damit die Membran 9 gegen das Gehäuseteil 1a zu drücken. Die Spule kann dadurch gewonnen werden, daß ausgehend von einem in der Achse der Kugelkalotte 11 liegenden zentralen Anfangsstück 12a spiralförmig die einzelnen Windungen bis zur äußersten Windung 12b auf die innere kugelkalottenförmige Fläche 13 des Gehäuseteiles 1a aufgewickelt wird. Diese so gebildete Leiteranordnung 12 stellt eine spiralförmige Flachspule dar, deren Anfangsstück 12a und Endwicklung 12b jeweils über die Anschlüsse 14 und 15 an eine Energiequelle angeschlossen werden, von der aus ein kurzzeitiger Stromstoß durch die Spule geschickt wird. Beim Ausführungsbeispiel geschieht dies dadurch, daß ein Kondensator 16 nach der Aufladung über eine Funkenstrecke entladen wird. Bei der Entladung des Kondensators 16 über die Funkenstrecke 17 fließt durch die Leiteranordnung 12 ein kurzzeitiger hoher Stromstoß. Dieser induziert in der Metallmembran 9 entgegengesetzte Ströme, die zur Abstoßung der Membran 9 und zur Abstrahlung einer intensiven Stoßwelle in die Flüssigkeit
im Hohlraum 2 führen. Diese Stoßwelle breitet sich aufgrund der
kugelkalottenförmigen Ausbildung der Leiteranordnung 12, der
eng daran anliegenden Isolierschicht 10 und der ebenfalls
kugelkalottenförmigen Metallmembran 9 in der Flüssigkeit im
Raum 2 und durch die Abdeckung und den Ansatzpuffer 3a durch
die Körperoberfläche 6 aus und gelangt auf das zu behandelnde
Konkrement 18 hin, das beim Ausführungsbeispiel ein Stein in
einer Niere 19 sein kann. Die Stoßwelle trifft daher im Fokus
20 der kugelkalottenförmigen Flächen 13 und 11 auf das Konkrement 18 auf und führt dort in bekannter Weise durch wiederholte Einwirkung zu einem Zerbrechen des Konkrementes. Anstelle
des Kondensators 16 und der Funkenstrecke 17 können auch andere
geeignete Anordnungen zur Erzeugung eines Stromstoßes verwendet
werden, so zum Beispiel ein gesteuerter Impulsgenerator, der
Stromstöße hoher Folgefrequenz abgeben kann.

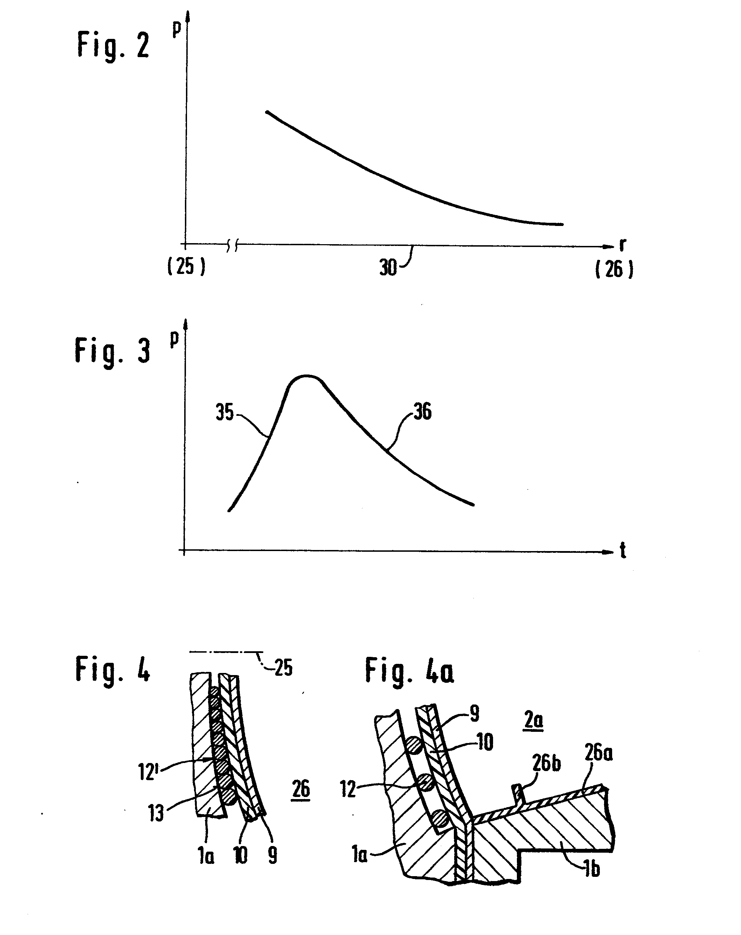

Schließlich ist in der Fig. 1 noch eine Achse 25 gekennzeichnet, die durch den Mittelpunkt der kugelkalottenförmigen
Fläche 11 bzw. 13 und den Fokus 20 verläuft sowie ein Außenbereich 26 angedeutet, der sich etwa im Bereich um die Mantelfläche des von der kugelkalottenförmigen Fläche 11 bzw. 13 und
dem Fokus 20 gebildeten Kegelstumpf befindet. Ebenfalls ist der
Fig. 1 noch zu entnehmen, daß bei der dargestellten Einrichtung
die Leiteranordnung 12 nicht aus dicht aneinanderliegenden Windungen besteht, sondern der Abstand der einzelnen Windungen
sich nach außen hin, also zum Rand der kugelkalottenförmigen
Fläche 11 bzw. 13 hin vergrößert. Durch diese Anordnung wird
eine Abschwächung des Profils der Druckamplitude der erzeugten
Stoßwelle von der Systemachse 25 hin zum Außenbereich 26
erreicht. Eine derartige Absenkung des Profils der Druckamplitude der Stoßwelle ist beispielhaft in der Fig. 2 dargestellt.

In der Fig. 2 ist ein Diagramm dargestellt, auf dessen Abszisse
der radiale Abstand r von der Systemachse 25 einer zum Beispiel
mittels der Anordnung gemäß Fig. 1 erzeugten Stoßwelle aufgetragen ist, während die Ordinate des Diagramms die Druckamplitude p der Stoßwelle darstellt. Da im Zusammenhang mit der Fig. 2 nur der Außenbereich der Stoßwelle von Interesse ist, ist in dem Diagramm der Fig. 2 auch nur dieser Außenbereich 30 gezeigt, was durch die Unterbrechung der Abszisse angedeutet sein soll. Dieser Außenbereich 30 entspricht dabei etwa dem Randbereich 26 der Fig. 1, während die Achse 25 dieser Fig. 1 dem Nullpunkt des Diagramms der Fig. 2 gleichgesetzt werden kann. Prinzipiell können jedoch die Ausführungen zur Fig. 2 auch auf den gesamten Bereich der Stoßwelle angewandt werden. Die in der Fig. 2 eingezeichnete Kurve 31 zeigt eine Absenkung des Profils der Druckamplitude, wie sie im Zusammenhang mit der Beschreibung der Fig. 1 schon erwähnt wurde. Die Kurve 31 kann dabei nicht nur den dargestellten kontinuierlichen Verlauf haben, sondern auch zum Beispiel einen stufenförmigen Verlauf. Der für den entsprechenden Anwendungsfall optimale Verlauf des örtlichen Druckverlaufs kann mit Hilfe von Versuchen und/oder Berechnungen herausgefunden werden. Durch eine derartige Druckamplitudenprofilabsenkung 31 im Randbereich 30 einer Stoßwelle wird einerseits erreicht, daß sich in diesem Randbereich keine Randbeugungswellen, und damit auch keine Unterdruckpulse bilden, sowie andererseits, daß die Ausgestaltung der gesamten Stoßwelle, insbesondere bezüglich ihres zeitlichen Verlaufs verbessert wird. Die Vermeidung von Unterdruckpulsen bewirkt dabei die Vermeidung von Gewebeschädigungen zum Beispiel im Bereich der behandelten Niere während die bessere Ausgestaltung der Stoßwelle gleichzeitig eine größere Wirksamkeit bei der Zerstörung des Konkrementes, zum Beispiel des Nierensteins verursacht.

Der Effekt, der durch eine Druckamplitudenprofilabsenkung gemäß der Fig. 2 erreicht wird, nämlich eine wesentliche Verringerung von Unterdruckpulsen und damit Gewebeverletzungen, kann dadurch noch wesentlich erhöht werden, daß der der Spulenanordnung 12 zugeführte Stromstroß so beeinflußt wird, daß er einen ganz bestimmten zeitlichen Druckverlauf hervorruft. Einen besonders vorteilhaften derartigen Druckverlauf zeigt die Fig. 3.

In der Fig. 3 ist ein Diagramm dargestellt, auf dessen Abszisse die Zeit t abgetragen ist, dessen Ordinate hingegen die Druckamplitude p einer Stoßwelle darstellt. Die in das Diagramm eingezeichnete Kurve umfaßt einen Druckamplitudenanstieg 35 und eine nachfolgende Druckamplitudenverringerung 36. Dabei ist der Druckamplitudenanstieg 35 wesentlich steiler ausgestaltet als die Druckamplitudenverringerung 36. Bei dem dargestellten Verlauf der Druckamplitude p über der Zeit t muß es sich nicht in jedem Fall um den optimalen Verlauf für eine Verringerung der Unterdruckpulse handeln. Es hat sich jedoch herausgestellt, daß eine zeitlich zuerst sehr schnelle Erhöhung und eine nachfolgende langsame Verringerung der Druckamplituden von Stoßwellen immer von Vorteil ist. Die optimale Form des zeitlichen Druckverlaufs kann bei dem entsprechenden Anwendungsfall mit Hilfe von Versuchen und/oder Berechnungen herausgefunden werden. Dabei kann die im Zusammenhang mit der Fig. 1 beschriebene Anordnung, die aus dem Kondensator 16 und der Funkenstrecke 17 besteht, durch einen Impulsgenerator ersetzt werden, mit dessen Hilfe nahezu beliebige elektrische Impulse zur Ansteuerung der Leiteranordnung 12 erzeugt werden können.

Bei der Einrichtung gemäß der Fig. 1 wird eine Druckamplitudenprofilabsenkung gemäß der Fig. 2 durch eine Verringerung der Wicklungsdichte der Leiteranordnung 12 erreicht. Eine derartige Absenkung des Profils der Druckamplitude kann jedoch auch auf andere Arten erreicht weden, so zum Beispiel mit Hilfe einer Ausgestaltung der Einrichtung der Fig. 1 gemäß der Fig. 4.

In der Fig. 4 liegt die Metallmembran 9 an der Isolierfolie 10 an, die ihrerseits über eine Leiteranordnung 12 mit dem Gehäuseteil 1a gekoppelt ist. Die Innenseite des Gehäuseteils 1a weist dabei eine kugelkalottenförmige Fläche 13 auf. Im Gegensatz zur Fig. 1, wo der zur Leiteranordnung 12 gewickelte Draht immer denselben Drahtdurchmesser besitzt, ändert sich bei der Fig. 4 diese Drahtstärke. So weist der der Achse 25 näherliegende Draht einen kleineren Durchmesser auf als ein weiter entfernt von der Achse 25 liegender Draht. Die Drahtstärke nimmt also von der Achse 25 aus nach außen hin zum Außenbereich 26 zu. Der Effekt einer derartigen nach außen hin zunehmenden Drahtstärke ist derselbe wie der schon erläuterte Effekt bei einer nach außen abnehmenden Wicklungsdichte. Besonders vorteilhaft ist es, als Leiteranordnung 12 statt einem Draht ein einen rechteckigen Querschnitt aufweisendes Leiterband zu verwenden, dessen Dicke in einfacher Weise bei seiner Herstellung, also zum Beispiel beim Auswalzen des Leiterbandes, beeinflußt werden kann. Dabei ist es auch möglich, das Merkmal der Fig. 1, nämlich die nach außen hin zunehmende Vergrößerung des Abstands der einzelnen Drähte der Leiteranordnung 12, mit dem Merkmal der Fig. 4, nämlich der nach außen hin zunehmenden Stärke der einzelnen Drähte der Leiteranordnung 12 zu kombinieren, wobei dabei statt der Leiterdrähte wieder auch Leiterbahnen verwendet werden können. Die Zwischenräume, die aufgrund der abnehmenden Windungsdichte entstehen, können dabei in vorteilhafter Weise durch Isoliermaterial, bei Leiterbahnen zum Beispiel durch entsprechende Isolierbahnen oder mittels breiterer Leiterdrähte bzw. Leiterbahnen der einzelnen Windungen ausgefüllt werden.

Eine weitere Möglichkeit, eine Druckamplitudenprofilabsenkung gemäß der Fig. 2 zu erhalten, besteht darin, die Leiteranordnung 12 aus Einzelwindungen oder Einzelspulen (12', 12", 12'", 12"", Fig. 12) herzustellen, die mit elektrischen Impulsen (1014 bis 1018) bestimmter Stärke und Zeitdauer beaufschlagbar sind. Dabei können die Einzelwindungen konzentrisch oder auch in anderer Art und Weise auf der kugelkalottenförmigen Fläche 13 angeordnet sind. Dabei hat es sich als besonders vorteilhaft erwiesen, bei konzentrisch angeordneten Einzelwindungen die der Systemachse 25 näherliegenden Spulen mit einem hohen Entladestrom zu beaufschlagen, wohingegen die weiter außenliegenden
Spulen mit einem niedrigeren Entladestrom. Dies kann zum Beispiel dadurch erreicht werden, daß die einzelnen Spulen durch
geeignete Serien- und/oder Parallelschaltungen von der den
Stromstoß erzeugenden Einrichtung angesteuert werden und/oder
verschiedene Drahtstärken aufweisen und/oder daß Spulenabschnitte durch herausgeführte Stichleitungen (Fig. 13) und
geeignete Nebenschlüsse mit unterschiedlicher Stromstärke
beaufschlagt werden. Ebenfalls ist es möglich, die Abstände der
Einzelwindungen variabel zu gestalten. Bei nicht konzentrisch
angeordneten Einzelspulen hat es sich als günstig erwiesen, im
Innenbereich der kugelkalottenförmigen Fläche 13 mehr Windungen
anzubringen als im Außenbereich. Die Außenform aller Einzelwindungen kann dabei auf verschiedene Arten gestaltet werden,
so zum Beispiel sternförmig, drei- oder mehreckig usw. Diese
Anordnung kann ebenfalls noch durch variable Drahtdicken
und/oder verschiedene Ansteuerimpulse der einzelnen Windungen
erweitert werden. Ebenfalls ist es nicht zwingend notwendig,
daß bei konzentrischen, wie auch bei nicht konzentrischen
Anordnungen die Form der einzelnen Windungen in jedem Fall
kreisförmig ist.

Ist bei einer Einrichtung gemäß der Fig. 1 zur besseren Ausbildung einer Stoßwelle der Hohlraum 2 kegelstumpfförmig ausgebildet, so hat es sich als besonders vorteilhaft herausgestellt, die Druckamplitudenprofilabsenkung gemäß der Fig. 2
dadurch zu erreichen, daß Änderungen am Kegelstumpf vorgenommen
werden. Bei derartigen Änderungen kann es sich beispielsweise
um eine in Richtung der Abdeckung 3 weisenden Aufweitung des
Kegelstumpfes handeln, oder um Abweichungen von der kreisförmigen Gestalt des Kegelstumpfes. Auch ist es möglich, die
Wanddämpfung des Kegelstumpfes zu verändern, indem zum Beispiel
Dämmaterial auf die Mantelfläche des Kegelstumpfes aufgebracht
wird. Schließlich kann die Druckamplitude der Stoßwelle bei
Verwendung eines Kegelstumpfes auch noch dadurch abgesenkt
werden, daß ausgehend von der Mantelfläche des Kegelstumpfes stiftähnliche Gebilde in den Hohlraum hineinragen, die dadurch eine Streuung der Stoßwelle verursachen. Auch bei der Verwendung eines kegelförmigen Hohlraums ist es möglich, die eben beschriebenen Maßnahmen mit den die Leiteranordnung 12 betreffenden Maßnahmen zu kombinieren.

Es wurde schon erwähnt, daß es möglich ist, eine Druckamplitudenprofilabsenkung gemäß der Fig. 2 dadurch zu erreichen, daß die Leiteranordnung 12 beispielsweise eine sternförmige Gestalt hat. Analog dazu ist es ebenfalls möglich, statt der Außenform der Leiteranordnung andere Bauteile der Einrichtung der Fig. 1 entsprechend zu verändern. So kann zum Beispiel die Abdeckung 3 oder der Hohlkörper 2, insbesondere bei Verwendung eines Kegelstumpfes als Hohlkörper, mit einer sternförmigen oder anderen, nicht kreisförmigen Gestalt versehen werden. Dabei ist es ebenfalls wieder möglich, auch drei-, vier- oder mehreckige Gebilde (Fig. 15) zu verwenden und diese mit den bisher beschriebenen Möglichkeiten zur Druckampitudenabsenkung zu kombinieren. Ebenso kann durch geeignete Dämpfungssysteme, Absorber oder Streukörper die Stoßwelle vom Rande her in der erforderlichen Weise abgeschwächt werden.

Bisher wurde die Erfindung im Zusammenhang mit der in der Fig. 1 dargestellten Einrichtung erläutert. Es ist jedoch auch möglich, die Erfindung im Zusammenhang mit Einrichtungen zu verwenden, die statt der kugelkalottenförmigen Anordnung der Metallmembran 9 eine ebene Metallmembran besitzen. Bei derartigen Einrichtungen muß dann die von der Metallmembran erzeugte Stoßwelle mit Hilfe von Reflektoren und Linsen auf das Konkrement fokussiert werden. Es können jedoch auch bei einer derartigen Einrichtung sämtliche bisher erläuterten Möglichkeiten zu einer Druckamplitutenprofilabsenkung gemäß der Fig. 2 entsprechend angewandt werden, so daß auch hier mit Hilfe der Erfindung die Bildung von Unterdruckpulsen und damit Gewebeverletzungen verhindert werden können.

Schließlich ist es auch möglich, statt der aus spulenförmigen Elementen bestehenden Leiteranordnung andere, die Stoßwelle erzeugende Quellen zu verwenden, so zum Beispiel punktförmige, linienförmige, gekrümmt linienförmige, flächenhafte, insbesondere kreisförmige oder gekrümmt flächenhafte Quellen. Die von diesen Quellen erzeugten Stoßwellen werden dann mit Hilfe von Reflektor- und/oder Linsenanordnungen auf das Konkrement fokussiert, wobei hierbei die Wellenführung auch noch mit Hilfe der Streuung, der Absorbtion oder anderer Arten der Wellenbeeinflussung verändert wird. Dabei können in besonders vorteilhafter Weise die zur Wellenführung, insbesondere zur Fokussierung verwendeten Anordnungen gleichzeitig auch zur Druckamplitudenprofilabsenkung benutzt werden. Als Quellen können zum Beispiel piezoelektrische oder magnetostriktive Elemente (Fig. 14) oder Flüssigkeitsfunkenentladungen Anwendung finden, mit denen die entsprechenden Quellenformen dann hergestellt werden.Die Beeinflussung der Stoßwelle, insbesondere deren Druckamplitudenveränderung im Randbereich der Stoßwelle, wird bei der Verwendung der zuletzt genannten Elemente dadurch erreicht, daß zum Beispiel die Ansteuerspannungen der einzelnen Elemente variiert werden, die Anordnungsdichte der einzelnen Elemente verändert wird sowie die Baugröße oder Bauform der einzelnen Elemente verschieden gestaltet wird.

Bei Systemen mit fokussierenden Anordnungen, also zum Beispiel mit Reflektoren und/oder Spiegeln, ist es zusätzlich oder alternativ möglich, die Stoßwelle im Hinblick auf eine Druckamplitudenveränderung dadurch zu beeinflussen, daß die Bauform und/oder die Baugröße der fokussierenden Anordnung verändert wird. Besonders vorteilhaft ist es dabei, die Anordnung, also zum Beispiel die Linse oder den Reflektor, sternförmig, drei- oder mehreckig, oder in einer ausgefransten Form auszugestalten. Ebenso ist natürlich eine Absenkung der Randamplitude durch sekundäre dämpfende, absorbierende oder streuende Maßnahmen möglich.

Allgemein kann also im Grunde genommen bei allen Systemen zur Erzeugung und Fokussierung von Stoßwellen deren Druckampitude bezüglich der örtlichen und zeitlichen Ausbreitung dadurch beeinflußt werden, daß die lokale Anregungsdichte und/oder die lokale Anregungsstärke und/oder die Bauform und/oder die Baugröße der die Stoßwelle erzeugenden Quelle oder die Bauform und/oder die Baugröße der fokussierenden Anordnung verändert wird und /oder die Dämpfung, Absorption oder Streuung der Stoßwelle während der Ausbreitung radial verändert wird.

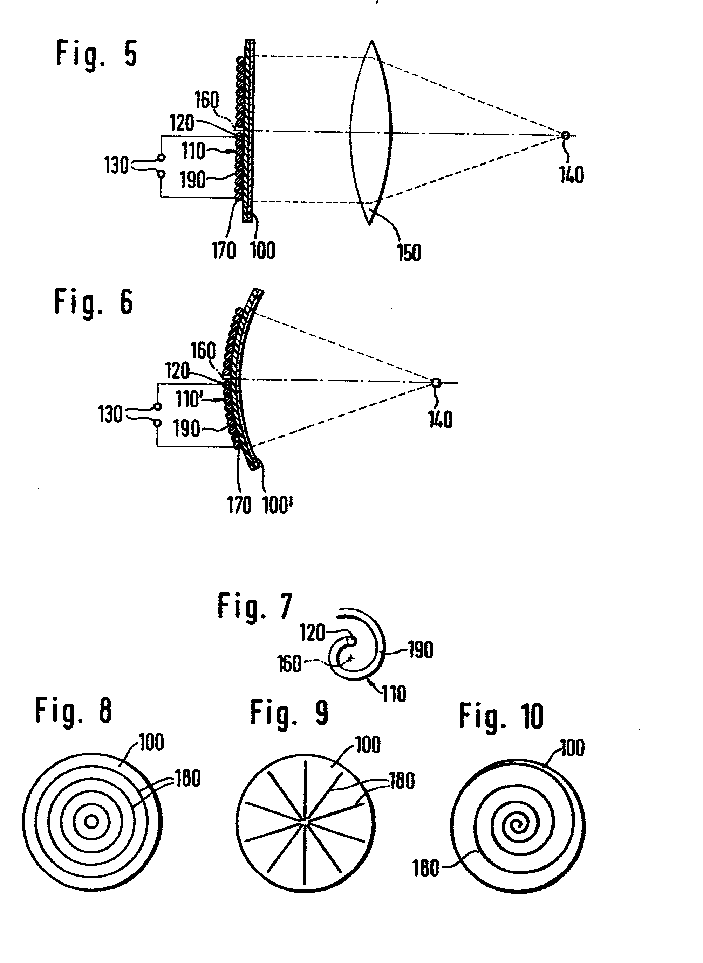

In den Fig. 5 und 6 ist in vereinfachter Darstellung noch einmal der prinzipielle Aufbau von elektromagnetischen Einrichtungen zur berührungsfreien Zertrümmerung von Konkrementen im Körper von Lebewesen schematisch dargestellt. Eine Metallmembran 100 grenzt an eine Spule 110 an, die aus einem spiralförmig gewickelten Leiter 190 besteht, dessen innerer Anfangspunkt 120 und äußeres Ende 170 zu den Spulenanschlüssen 130 führen. Das Zentrum der Spule ist mit der Bezugsziffer 160 gekennzeichnet und entspricht etwa dem Zentrum der im wesentlichen kreisförmigen Metallmembran 100. Wird die Spule 110 an ihren Anschlüssen 130 von einem Stromimpuls beaufschlagt, so bewirkt dies eine Abstoßung der Membran 100 von der Spule 110. Da sich bei speziellen Ausgestaltungen derartiger Einrichtungen auf beiden Seiten der Membran-Spulen-Anordnung 100,110 weitere Körper befinden und insbesondere die Membran 100 fest eingespannt ist, kann dadurch der durch die Abstoßung der Membran 100 von der Spule 110 erzeugte Stoß weitergegeben und durch entsprechende Einrichtungen auf das zu zertrümmernde Konkrement fokussiert werden.

Bei der Fig. 5 handelt es sich um eine ebene Metallmembran 100, die an eine ebenfalls ebene Spule 110 angrenzt. Dadurch ergibt sich bei Beaufschlagung der Spule mit einem Stromimpuls zuerst eine ebene Stoßwelle, die mit Hilfe einer Linse 150 auf einen Fokus 140 konzentriert wird, in dem sich ebenfalls das zu zertrümmernde Konkrement befindet. Demgegenüber ist in der Fig. 6 eine kugelkalottenförmige Membran 100' gezeigt, die an einer ebenfalls kugelkalottenförmigen Spule 110' anliegt, und die dadurch bei Beaufschlagung der Spule 110' mit einem Stromimpuls eine Stoßwelle erzeugt, die sich ohne die Hilfe weiterer Fokussiereinrichtungen im Fokus 140 konzentriert, in dem sich wiederum das zu zerstörende Konkrement befindet.

Der Fig. 5, wie auch der Fig. 6, ist zu entnehmen, daß der innere Anfangspunkt 120 des Leiters 190 nicht im Zentrum 160 der Spule 110 bzw. 110' liegt, sondern außerhalb dieses Zentrums. Dieser Sachverhalt ist in der Fig. 7 nochmals dargestellt.

Fig. 7 zeigt eine schematische Draufsicht auf die Spule 110 der Fig. 5 oder der Fig. 6. Dabei sind die einzelnen Windungen der Spule 110 zum Zwecke der besseren Darstellung nicht als aneinanderanliegend dargestellt, sondern mit einem Abstand gezeichnet. Ebenfalls ist nur die erste Windung der Spule 110 gezeigt, die den inneren Anfangspunkt 120 des Leiters 190 aufweist und sich direkt um das Spulenzentrum 160 windet. Wichtig ist, daß der Spulenanfang 120 außerhalb des Spulenzentrums 160 liegt.

Mit Hilfe dieser erfindungsgemäßen Anordnung des Spulenanfangs 120 wird erreicht, daß der Krümmungsradius des Leiters 190 keine zu kleinen Werte annimmt und dadurch die Isolierung der Spule 110 nicht bricht. Ebenfalls werden dadurch scharfe Ecken und Kanten des Leiters 190 vermieden, so daß insgesamt die Durchschlags- bzw. Kurzschlußgefahr bei Beaufschlagung der Spule 110 mit Stromimpulsen wesentlich verringert, also die Zuverlässigkeit und die Lebensdauer der Spule erhöht wird. Des weiteren ist es mit Hilfe der erfindungsgemäßen Anordnung des inneren Spulenanfangs 120 außerhalb des Spulenzentrums 160 möglich, die Spule maschinell herzustellen, da die schwierigen, bisher von Hand durchzuführenden Wickelarbeiten im Zentrum der Spule entfallen.

Die Fig. 8, 9 und 10 zeigen schematisch drei Ausgestaltungsmöglichkeiten der Metallmembran 100 bzw. 100' der Fig. 5 und 6.
Dabei sind in den Fig. 8, 9 und 10 Dehnungsfalten mit der
Bezugsziffer 180 gekennzeichnet. Diese Dehnungsfalten sind
dabei in der Fig. 8 kreisförmig angeordnet, in der Fig. 9
radial sowie schließlich in der Fig. 10 spiralförmig. Die
Membran 100 der Fig. 8, 9 und 10 ist kreisförmig dargestellt.

Die Dehnungsfalten 180 der Fig. 8 bis 10 können auf mehrere
Arten hergestellt werden. So ist es möglich, durch entsprechendes wellenförmiges Falten die gezeigten Strukturen herzustellen, ebenfalls ist es denkbar, mit Hilfe verschiedener Materialdicken der Metallmembran 100 die Dehnungsfalten 180 zu
erhalten. Im Zusammenhang mit der letzten Möglichkeit ist es
besonders vorteilhaft, die Membran 100 aus Folienschichtpaketen
aufzubauen.

Mit Hilfe der in den Fig. 8 bis 10 dargestellten Dehnungsfalten
180 wird erreicht, daß bei Beaufschlagung der Spule 110 mit
einem Stromimpuls und der dadurch entstehenden Abstoßung der
Membran 100 von der Spule 110 die Belastung der Membran 100
gering gehalten wird, gleichzeitig aber eine Ausdehnung der
Membran 100 möglich ist. Es wird also einerseits die Membran
vor zu starker Beanspruchung und damit vor Brüchen geschützt,
gleichzeitig wird die Ausbildung der durch die Membran 100
erzeugten Stoßwellen unterstützt. Insbesondere wird mittels der
Dehnungsfalten 180 die Lebensdauer der Membran 100 wesentlich
erhöht, da die bisher vor allen im Zentrum der Membran 100
vorhandenen hohen Beanspruchungen der Membran 100 durch die
Dehnungsfalten 180 wesentlich verringert werden.

Abschließend sei nochmals darauf hingewiesen, daß sich die Erfindung nicht ausschließlich auf ebene Membran-Spulen-Anordnungen gemäß der Fig. 5 beschränkt, sondern ebenfalls bei entsprechenden anderen Anordnungen, zum Beispiel bei kugelkalottenförmigen Anordnungen gemäß der Fig. 6, oder auch Fig. 1 anwendbar ist. Ebenfalls ist es möglich, statt des in den Fig. 5 und 6 gezeigten Leiters mit rundem Querschnitt ein entsprechendes Leiterband mit rechteckigem Querschnitt zu verwenden, das zum Beispiel zur Ausbildung einer kugelkalottenförmigen Form entsprechend ausgedreht bzw. ausgefräst wird. Auch ist die Erfindung, wie schon erwähnt wurde, nicht nur auf kreisförmige Anordnungen beschränkt, sondern in entsprechender Weise auch auf zum Beispiel sternförmige oder mehreckige Anordnungen anwendbar.

Ein besonders bevorzugtes Anwendungsgebiet der Erfindung ist die berührungsfreie Zertrümmerung von Nierensteinen im Körper von Lebewesen, insbesondere von Menschen, die anhand der Fig. 1 bis 4 erläutert ist.

## Ansprüche

1. Verfahren zur berührungsfreien Zertrümmerung von Konkrementen im Körper von Lebewesen unter Zuhilfenahme von Stoßwellen, die auf das Konkrement fokussiert werden, dadurch gekennzeichnet, daß das Profil der Druckamplituden (p) der Stoßwellen örtlich (r) nach außen hin abgesenkt (31) wird.

2. Verfahren nach Anspruch 1, dadurch gekennzeichnet, daß die Druckamplituden (p) der Stoßwellen zeitlich (t) zuerst schnell erhöht (35) und dann wieder langsamer verringert (36) werden.

3. Verfahren nach Anspruch 1 oder 2, dadurch gekennzeichnet, daß zur Beeinflussung des Profiles der Druckamplituden (p) auf die Anregungsdichte der Stoßwellen eingewirkt wird.

4. Verfahren nach einem der Ansprüche 1 bis 3, dadurch gekennzeichnet, daß zur Beeinflussung der Druckamplituden (p) auf die Anregungsstärke der Stoßwellen eingewirkt wird.

5. Einrichtung zur Durchführung des Verfahrens nach einem der Ansprüche 1 bis 4, dadurch gekennzeichnet, daß erste Mittel (12) vorgesehen sind für eine örtlich (r) nach außen hin verlaufende Absenkung (31) des Druckamplitudenprofiles (p) der Stoßwellen.

6. Einrichtung nach Anspruch 5, dadurch gekennzeichnet, daß
zweite Mittel (17) vorgesehen sind für eine zeitlich (t)
zuerst schnelle Erhöhung (35) und darauffolgende Verringerung (36) der Druckamplituden (p) der Stoßwellen.

7. Einrichtung nach Anspruch 5 oder 6, dadurch gekennzeichnet, daß die ersten Mittel (12) wenigstens ein von
elektrischen Impulsen beaufschlagbares Spulenelement zur
Erzeugung der Stoßwellen umfassen.

8. Einrichtung nach Anspruch 5 oder 6, dadurch gekennzeichnet, daß die ersten Mittel (12) wenigstens ein von elektrischen Impulsen beaufschlagbares piezoelektrisches Element zur Erzeugung der Stoßwellen umfassen.

9. Einrichtung nach Anspruch 5 oder 6, dadurch gekennzeichnet, daß die ersten Mittel (12) wenigstens ein von
elektrischen Impulsen beaufschlagbares magnetostriktives
Element zur Erzeugung der Stoßwellen umfassen.

10. Einrichtung nach Anspruch 5 oder 6, dadurch gekennzeichnet, daß die ersten Mittel (12) wenigstens ein die Stoßwelle fokussierendes Element umfassen.

11. Einrichtung nach einem der Ansprüche 7 bis 10, dadurch
gekennzeichnet, daß die Bauform der ersten Mittel (12)
mehreckig, insbesondere sternförmig ist.

12. Einrichtung nach Anspruch 6, dadurch gekennzeichnet, daß
die zweiten Mittel (17) wenigstens eine an eine Spannung
anlegbare Funkenstrecke zur Erzeugung von elektrischen
Impulsen umfassen.

13. Einrichtung nach Anspruch 6, dadurch gekennzeichnet, daß die zweiten Mittel (17) wenigstens einen an eine Versorgungsspannung angeschlossenen Impulsgenerator zur Erzeugung von elektrischen Impulsen umfassen.

14. Einrichtung nach einem der Ansprüche 7 bis 10 und Anspruch 12 oder 13, dadurch gekennzeichnet, daß die von den zweiten Mitteln (17) erzeugten elektrischen Impulse die ersten Mittel (12) beaufschlagen.

15. Einrichtung nach Anspruch 5, dadurch gekennzeichnet, daß zur Erzeugung der Stoßwellen eine an ein eine Gegenkraft ausübendes Übertragungsmedium angrenzende Metallmembran vorgesehen ist, die durch eine dünne Isolierschicht von einer auf der Stirnseite eines Trägers aufgebrachten flachen und spiralförmigen Leiteranordnung getrennt ist, die mit einem elektrischen Impuls bestimmter Stärke und Zeitdauer beaufschlagbar ist, und eine nach außen hin abnehmende Windungsdichte besitzt, bei der ferner die Leiteranordnung (12) auf ihrer an die Metallmembran angrenzenden Seite zu einer Kugelkalottenfläche ausgestaltet ist und eine nach außen hin abnehmende Windungsdichte besitzt, und bei der an diese Kugelkalottenfläche die Metallmembran unter dem Druck des Übertragungsmediums angedrückt ist.

16. Einrichtung nach Anspruch 5, dadurch gekennzeichnet, daß zur Erzeugung der Stoßwellen eine an ein eine Gegenkraft ausübendes Übertragungsmedium angrenzende Metallmembran vorgesehen ist, die durch eine dünne Isolierschicht von einer auf der Stirnseite eines Trägers aufgebrachten flachen und spiralförmigen Leiteranordnung getrennt ist, die mit einem elektrischen Impuls bestimmter Stärke und Zeitdauer beaufschlagbar ist, bei der ferner die Leiteranordnung (12) auf ihrer an die Metallmembran angrenzenden Seite zu einer Kugelkalottenfläche ausgestaltet ist und eine nach außen hin zunehmende Leiterstärke besitzt, und bei der an diese Kugelkalottenfläche die Metallmembran unter dem Druck des Übertragungsmediums angedrückt ist.

17. Einrichtung nach Anspruch 5, dadurch gekennzeichnet, daß zur Erzeugung der Stoßwellen eine an ein eine Gegenkraft ausübendes Übertragungsmedium angrenzende Metallmembran vorgesehen ist, die durch eine dünne Isolierschicht von einer auf der Stirnseite eines Trägers angebrachten flachen Leiteranordnung getrennt ist, bei der ferner die Leiteranordnung (12) auf ihrer an die Metallmembran angrenzenden Seite zu einer Kugelkalottenfläche ausgestaltet ist und bei der an diese Kugelkalottenfläche die Metallmembran unter dem Druck des Übertragungsmediums angedrückt ist, wobei die Leiteranordnung (12) aus Einzelwindungen oder Einzelspulen besteht, die mit elektrischen Impulsen bestimmter Stärke und Zeitdauer beaufschlagbar sind.

18. Membran-Spulen-Anordnung für eine elektromagnetische Einrichtung zur berührungsfreien Zertrümmerung von Konkrementen im Körper von Lebewesen, mit einer Metallmembran sowie mit einer daran angrenzenden, aus einem spiralförmig aufgewickelten Leiter bestehenden Spule, insbesondere nach einem der Ansprüche 15 bis 17, dadurch gekennzeichnet, daß der innere Anfangspunkt (12) des Leiters (19) außerhalb des Zentrums (16) der Spule (11) liegt.

19. Anordnung nach Anspruch 18, dadurch gekennzeichnet, daß die Membran (10) Dehnungsfalten (18) besitzt.

20. Anordnung nach Anspruch 19, dadurch gekennzeichnet, daß die Dehnungsfalten (18) kreisförmig angeordnet sind.

21. Anordnung nach Anspruch 19, dadurch gekennzeichnet, daß die Dehnungsfalten (18) radial angeordnet sind.

22. Anordnung nach Anspruch 19, dadurch gekennzeichnet, daß die Dehnungsfalten (18) spiralförmig angeordnet sind.

23. Anordnung nach einem der Ansprüche 19 bis 22, dadurch gekennzeichnet, daß die Dehnungsfalten (18) wellenförmig ausgebildet sind.

24. Anordnung nach einem der Ansprüche 18 bis 23, dadurch gekennzeichnet, daß die Membran (10) aus Folienschichtpaketen aufgebaut ist.

25. Anordnung nach Anspruch 5, dadurch gekennzeichnet, daß als die ersten Mittel akustische Dämpfungssysteme, Absorptionssysteme oder Streusysteme vorgesehen sind, die im Ausbreitungsweg der Stoßwelle angeordnet sind.

Fig. 1

**Fig. 2**

(25)      30      (26)

**Fig. 3**

35     36

**Fig. 4**

25

12'   13   1a   10   9   26

**Fig. 4a**

9   10   2a   26b   26a   12   1a   1b

0209053

3/5

Fig. 5

160
120
110
130
190

170  100

150

140

Fig. 6

160
120
110'
130
190

170  100'

140

Fig. 7

120
160
190
110

Fig. 8

100
180

Fig. 9

100
180

Fig. 10

100
180
180

Fig. 11

12

Fig. 12

1014

1016

1017

1018

1015

12'

12''

12'''

12''''

Fig. 13

14

15

12'

12''

12'''

12''''

Fig. 14

U/I

t

t

t

t

t

Fig. 15